# EUROPEAN PATENT SPECIFICATION

(11) **EP 2 224 880 B1**
(45) Date of publication and mention of the grant of the patent: **16.01.2013**
(21) Application number: 08868366.9
(22) Date of filing: 24.12.2008
(51) Int. Cl.: A61F 2/06

(54) **IMPLANTABLE DEVICE**
IMPLANTIERBARE VORRICHTUNG
DISPOSITIF IMPLANTABLE

(30) Priority: 27.12.2007 US 16994 P
(43) Date of publication of application: 08.09.2010
(73) Proprietor: Cook Medical Technologies LLC, Bloomington, IN 47404 (US)
(72) Inventor: KUPPURATHANAM, Shyam, Bloomington IN 47403 (US); CHARLEBOIS, Steven, J., West Lafayette, IN 47906 (US); DIERKING, William, K., Louisville, KY 40222 (US); GREWE, David, D., West Lafayette IN 47906 (US)
(74) Representative: Ellis, Katherine Elizabeth Sleigh
(86) International application number: PCT/US2008/014035
(87) International publication number: WO 2009/085281

(56) References cited:
- EP-A- 1 543 798
- WO-A-92/03107
- US-A1- 2005 240 261
- US-A1- 2006 009 835
- US-B1- 6 652 571

## Description

### Technical Field

The present invention relates generally to an implantable device and methods of making the device. In particular, the invention relates to a device having a composite weave graft where the device is a vascular graft device.

### Background of the Invention

Aneurysms occur in blood vessels in locations where, due to age, disease or genetic predisposition, the blood vessel strength or resiliency is insufficient to enable the blood vessel wall to retain its shape as blood flows therethrough, resulting in a ballooning or stretching of the blood vessel at the limited strength/resiliency location to thereby form an aneurysmal sac. If the aneurysm is left untreated, the blood vessel wall may continue to expand, to the point where the remaining strength of the blood vessel wall is below that necessary to prevent rupture, and the blood vessel will fail at the aneurysm location, often with fatal result.

To prevent rupture, a stent graft of a tubular construction may be introduced into the blood vessel, for example, intraluminally. Typically, the stent graft is deployed and secured in a location within the blood vessel such that the stent graft spans the aneurysmal sac. The outer surface of the stent graft, at its opposed ends, is sealed to the interior wall of the blood vessel at a location where the blood vessel wall has not suffered a loss of strength or resiliency. Blood flow in the vessel is thus channelled through the hollow interior of the stent graft, thereby reducing, if not eliminating, any stress on the blood vessel wall at the aneurysmal sac location. Therefore, the risk of rupture of the blood vessel wall at the aneurysmal location is significantly reduced, if not eliminated, and blood can continue to flow through to the downstream blood vessels without interruption. An example of a stent graft for the treatment of aneurysms can be found in EP 1543798.

In many cases, however, a relatively smaller sized profile is needed or at least preferred for optimal delivery of a device through the vasculature. For example, in the case of the abdominal aorta, the device is introduced through narrow and tortuous anatomy, including the femoral and the iliac arteries, to implant the device and may be guided to branch vessels including the celiac, mesenteric, and renal arteries, which lead to various other body organs.

### Summary of the Invention

Preferred embodiments of the present invention generally provide an implantable graft device that has a reduced profile for delivery of the device through the anatomy of a patient.

According to a first aspect of the present invention, there is provided an implantable graft device having a composite weave graft as described in claim 1. The device comprises a graft body forming a lumen defining a longitudinal axis and comprising proximal and distal ends. The graft body comprises a composite of low dernier yarns and polymeric yarns configured for low profile delivery and radial elongation relative to the longitudinal axis during use. The graft body has a first portion and a second portion extending from the first portion. The first portion comprises at least one expandable stent radially attached thereto for support and the second portion having corrugations for enhanced kink resistance.

According to a second aspect of the present invention, there is provided an implantable prosthesis for treatment of a main vessel defect near one of or more branch vessels. The prosthesis comprises a graft body forming a lumen defining a longitudinal axis and comprising proximal and distal ends. The graft body comprises a composite of reduced diameter and elastic yarns configured for low profile delivery and radial elongation relative to the longitudinal axis during use. The graft body has a first portion and a second portion extending from the first portion. The first portion comprises at least one expandable stent radially attached thereto for support and the second portion has corrugations for enhanced kink resistance. The prosthesis further comprises an anchor portion extending from the proximal end of the graft body. The anchor portion has a first woven portion and a barb stent attached thereto for reduced migration of the graft device. The first woven portion is comprised of woven yarn. The reduced diameter yarn of the graft body has a smaller diameter than the yam of the anchor portion. The prosthesis further comprises an end portion extending from the distal end of the graft body. The end portion has a second woven portion and an expandable stent attached thereto. The second woven portion is comprised of woven yarn. The reduced diameter yarn of the graft body has a smaller diameter than the yam of the end portion.

According to a third aspect of the present invention, there is provided a method for making an implantable graft device having a composite weave graft as described in claim 7. The method comprises forming a graft body having a lumen defining a longitudinal axis and comprising proximal and distal ends defining a woven fabric having warp yarns aligned in a first direction and weft yarns aligned in a second direction and inner woven with the warp yarns. The woven fabric comprises a composite of reduced diameter and elastic yarns configured for low profile delivery and radial elongation relative to the longitudinal axis during use. The graft body has a first portion and a second portion extending from the first portion. The method further comprises attaching at least one expandable stent radially about the first portion for support and forming corrugations on the second portion for enhanced kink resistance.

Further objects, features, and advantages of preferred embodiments of the present invention will become apparent from consideration of the following description, provided by way of example only, and the appended claims when taken in connection with the accompanying drawings.

### Brief Description of the Drawing

Figure 1 a is a side view of an implantable graft device;
Figure 1b is an enlarged view of the device of Figure 1a in circle 1b;
Figure 2 is an environmental view of the implantable graft device of Figure 1; and
Figure 3 is a flow chart of one method for making an implantable graft device in accordance with another example of the present invention.

### Detailed Description

Embodiments of the present invention provide an implantable graft device having a composite weave graft. In one embodiment, the graft device comprises a graft body that comprises a composite of reduced diameter yarns and elastic yarns configured for low profile delivery and radial elongation relative to a longitudinal axis thereof.

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention. The materials, methods, and examples disclosed herein are illustrative only and not intended to be limiting.

The term "implantable" refers to an ability of a medical device to be positioned at a location within a body, such as within a body lumen.

As used herein, the term "body vessel" means any tube-shaped body passage lumen that conducts fluid, including but not limited to blood vessels such as those of the human vasculature system, oesophageal, intestinal, billiary, urethral and ureteral passages.

The term "branch vessel" refers to a vessel that branches off from a main vessel. The "branch vessels" of the thoracic and abdominal aorta include the celiac, inferior phrenic, superior mesenteric, lumbar, inferior mesenteric, middle sacral, middle suprarenal, renal, internal spermatic, ovarian (in the female), innominate, left carotid, and left subclavian arteries. As another example, the hypogastric artery is a branch vessel to the common iliac, which is a main vessel in this context. Thus, it should be seen that "branch vessel" and "main vessel" are relative terms.

The terms "about" or "substantially" used with reference to a quantity includes variations in the recited quantity that are equivalent to the quantity recited, such as an amount that is insubstantially different from a recited quantity for an intended purpose or function.

The term "stent" means any device or structure that adds rigidity, expansion force, or support to a prosthesis.

The term "stent graft" as used herein refers to a prosthesis comprising a stent and a graft material associated therewith that forms a lumen through at least a portion of its length.

The term "biocompatible" refers to a material that is substantially nontoxic in the *in vivo* environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, haemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

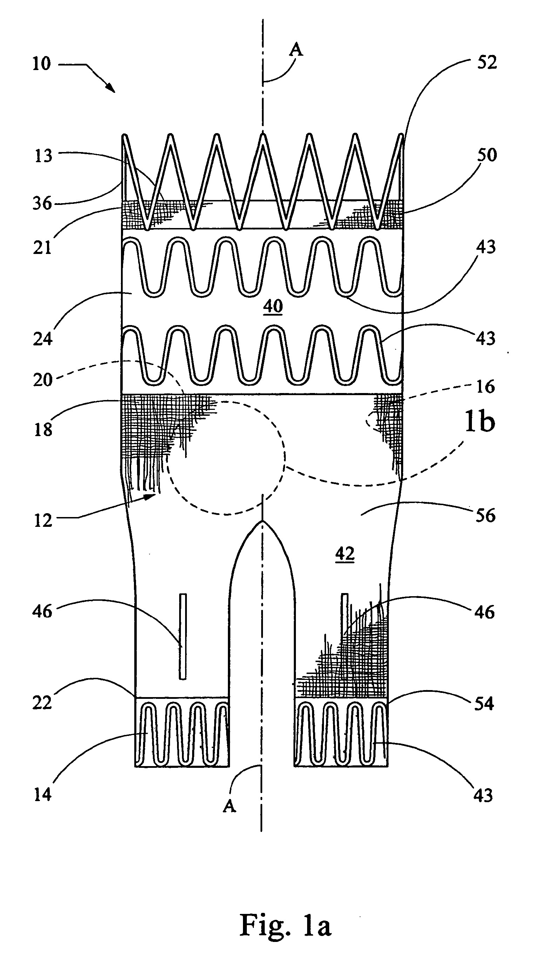

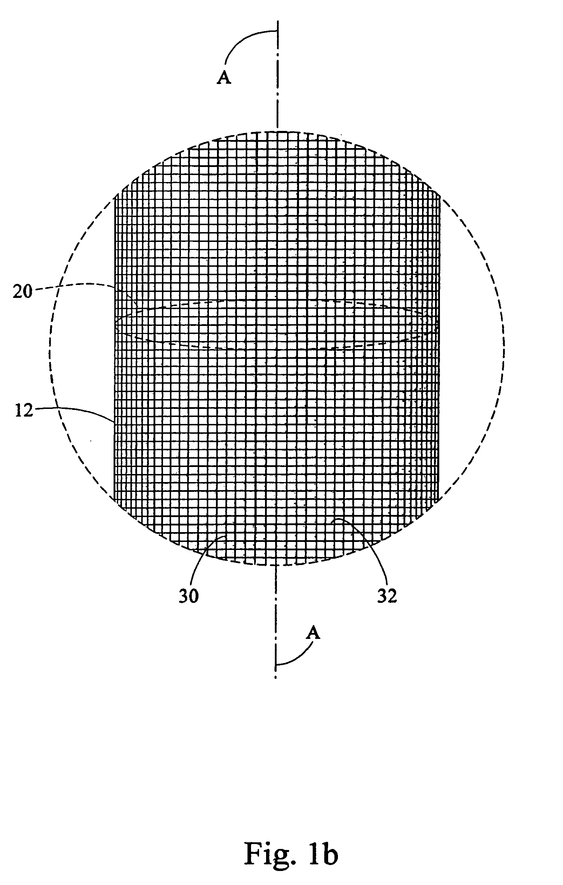

Figure 1 illustrates an implantable graft device 10 having a composite weave graft in accordance with an embodiment of the present invention. As shown, the device 10 comprises a graft body 12, an anchor portion 13 extending proximally therefrom, and an end portion 14 extending distally from the graft body 12. As further shown, the graft body 12 preferably comprises an inner side 16 and an outer side 18. The inner side 16 of the graft body 12 forms a lumen 20 defining a longitudinal axis and comprising proximal end 21 and distal end 22. In this embodiment, the graft body 12 comprises a composite of reduced diameter yam and elastic yam configured for low profile delivery and radial elongation relative to the longitudinal axis A during use. The yarn used in the present invention may be any suitable yarn. For example, in this embodiment, the reduced diameter yarn is low dernier polyester yam, preferably between about 0.5 and 1.5 denier and the elastic yarn may be any suitable polymeric or elastic yarn known in the art comprising a polymer, polyolefin, polyurethane, polyester, or polyamide.

In this embodiment, the graft body 12 comprises a woven fabric 24 having warp yarns 30 aligned in a first direction and weft yarns 32 aligned in a second direction. Preferably, the warp yarns 30 are the lengthwise threads attached to a loom before weaving begins. The weft yarns 32 (also known as woof or fill yarns) are the yarns that are shuttled back and forth across the warp yams 30, defining the woven fabric 24. Each of the warp and weft yarns 32 may be a thread of spun fibre. In this embodiment, the reduced diameter yarn is low dernier polyester fibre, preferably between about 0.5 and 1.5 denier. The fibre may be comprised of a various material (discussed in greater detail below). In one embodiment, the elastic yarns may be the weft yarn and the reduced diameter yarns may be the warp yarns 30. In another embodiment, the warp yarns 30 of the graft body 12 are reduced diameter yarns only.

As shown, the graft body 12 has a first portion 40 and a second portion 42 that extends from the first portion 40. In this embodiment, the first portion 40 comprises at least one expandable stent 43 radially attached thereabout for support and the second portion 42 has corrugations 46 for enhanced kink resistance. The expandable stent 43 may the be attached about the first portion 40 by any suitable means as discussed below.

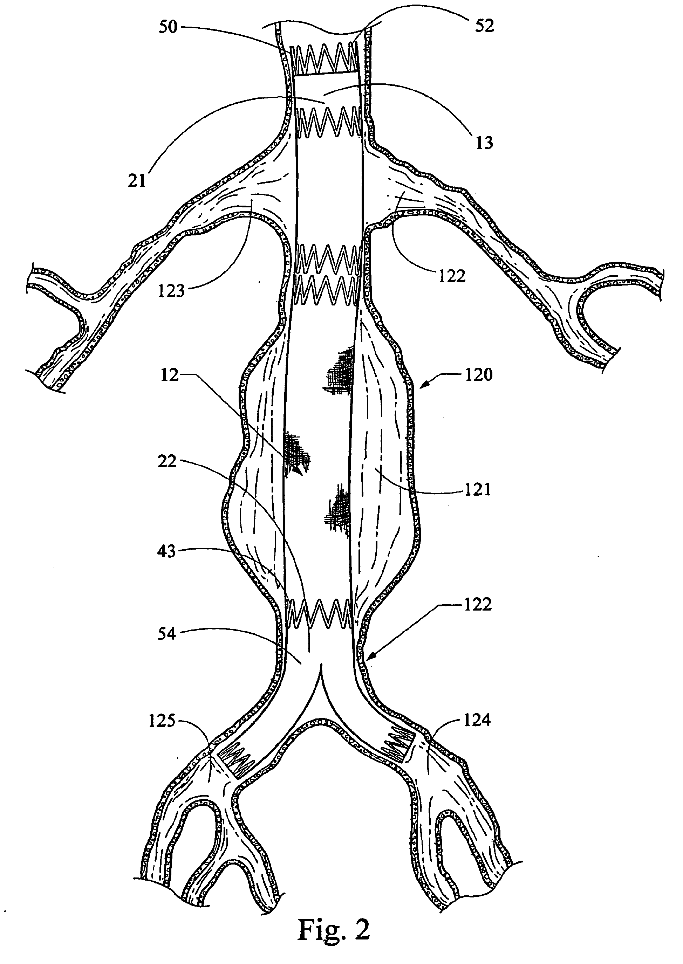

As shown in Figures 1 and 2, the anchor portion 13 extends from the proximal end 21 of the graft body 12. Preferably, the anchor portion 13 has a first woven portion 50 and a barb stent 52 attached thereto for reduced migration of the graft device 10. In this embodiment, the first woven portion 50 is comprised of woven yarn. The reduced diameter yarn of the graft body 12 has a smaller diameter than the yarn of the anchor portion 13. As shown, the first woven portion 50 comprises an inner side 16 to which the barb stent 52 is attached by any suitable means. In this embodiment, the barb stent 52 comprises a plurality of loops that are configured to be attached by sewing to the inner side 16 of the first woven portion 50.

In this embodiment, the end portion 14 extends from the distal end 22 of the graft body 12. As shown, the end portion 14 has a second woven portion 56 and an expandable stent 43 attached thereto. Preferably, the expandable stent 43 is attached to the inner side 16 of the graft device 10. The second woven portion 56 is also preferably comprised of woven yarn. Preferably, the reduced diameter yam of the graft body 12 has a smaller diameter than the yam of the end portion 14.

Figure 2 illustrates the aortic stent graft device 10 implanted within the renal arteries 122, 123 and the iliac arteries 124, 125 in accordance with one embodiment of the present invention. The aorta 120 has an aneurysm 121 between the renal arteries 122, 123 and the iliac arteries 124, 125. Though the above embodiments illustrate grafts located within the aorta, prostheses of the present invention may be implanted in any body vessel, including main vessels in which one or more branch vessels may be located.

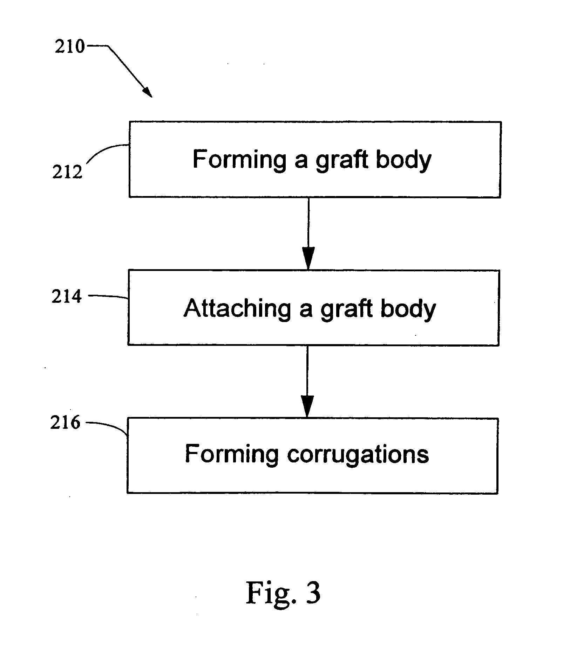

Figure 3 is a flow chart depicting a method 210 for making the implantable graft device 10 having a composite weave graft in accordance with one example of the present invention. In this example, the method 210 comprises forming in Box 212 the graft body 12 (discussed above). As mentioned, the graft body is formed with a lumen 20 to define a longitudinal axis. In this example, the graft body 12 has proximal end 21 and distal end 22 defining a woven fabric 24 having warp yarns 30 aligned in a first direction and weft yarns 32 aligned in a second direction and inner woven with the warp yarns 30. The woven fabric 24 comprises a composite of reduced diameter and elastic yarns configured for low profile delivery and radial elongation relative to the longitudinal axis during use. The graft body 12 has a first portion 40 and a second portion 42 extending from the first portion 40.

As shown, the method 210 further comprises in Box 214 attaching at least one expandable stent 43 radially about the first portion 40 for support and forming corrugations 46 in Box 216 on the second portion 42 for enhanced kink resistance. The stent 43 may be attached about the first portion 40 by any suitable means discussed herein. Then the anchor portion 13 is attached to the proximal end 21 and extends therefrom. As shown, the anchor portion 13 has a first woven portion 50 comprised of woven yarn. Preferably, the reduced diameter yam of the graft body 12 has a smaller diameter than the yarn of the first woven portion 50. As in Figure 1, the first woven portion 50 has a barb stent 52 attached thereto for reduced migration of the graft device 10.

The end portion 14 is then attached to the distal end 22 by any suitable means, i.e., sewing or stitching, and extends therefrom. As shown, the end portion 14 has a second woven portion 56 and an expandable stent 43 attached thereto. The second woven portion 56 is comprised of woven yarn. As mentioned above, the reduced diameter yarn of the graft body 12 has a smaller diameter than the yam of the end portion 14.

### Graft Weaves

The graft may comprise any kind of suitable weave or weaves. For example, the graft body may include, but is not limited to, weaves such as plain weaves, modified plain weaves, basket weaves, rep or rib weaves, twill weaves (e.g., straight twill, reverse twill, herringbone twill), modified twill weaves, satin weaves, double weaves (e.g., double-width, tubular double weave, reversed double weave), and any other related weaves. In one embodiment, the graft body comprises a plain weave having 150 ends per inch (2.54cm) and 250 picks per inch (2.54cm). An "end" refers to an individual warp yarn, and "sett" is the number of warp yarns per inch in a woven fabric. A "pick" refers to an individual weft yarn, and "pick count" is the number of weft yarns per inch (2.54cm) in a woven fabric.

### Graft Material

The graft material may comprise any biocompatible material suitable for weaving. The graft material may be natural, synthetic, or manufactured. For example, biocompatible materials include, but are not limited to, polyesters, such as poly(ethylene terephthalate); fluorinated polymers, such as polytetrafluoroethylene (PTFE) and fibres of expanded PTFE; and polyurethanes. In addition, materials that are not inherently biocompatible may be subjected to surface modifications in order to render the materials biocompatible. Examples of surface modifications include graft polymerisation of biocompatible polymers from the material surface, coating of the surface with a crosslinked biocompatible polymer, chemical modification with biocompatible functional groups, and immobilisation of a compatibilising agent such as heparin or other substances. Thus, any fibrous material may be used to form a graft body, provided the final textile is biocompatible.

Polymeric materials suitable for weaving graft material include polyethylene, polypropylene, polyaramids, polyacrylonitrile, nylons and cellulose, in addition to polyesters, fluorinated polymers, and polyurethanes as listed above. Desirably, the graft body material comprises one or more polymers that do not require treatment or modification to be biocompatible. More desirably, the graft body material comprises biocompatible polyesters. Even more desirable, graft body material comprises polyethylene terephthalate and PTFE. A preferred commercial example of polyethylene terephthalate especially suitable for weaving is Dacron^{™}. These materials are relatively inexpensive, easy to handle, have good physical characterstics and are suitable for clinical application.

The graft material may be woven of a single material or combination of materials. Determination of which combination of materials woven in which direction of the graft body that is most appropriate may be based on the type of clinical application, properties of the graft body that are desired, and further factors such as the weave type, yarn properties such as the size or denier of the yarn, finishing techniques, and/or permeability of the textile. For example, for percutaneous application, thin graft body are preferred. Such thin grafts comprise yarns that are fine or have a low denier. Desirably, graft body yarns range in size from about 0.1 denier to about 200 denier.

### Stents

One or more stents may be attached or adhered to the graft body by any means known to one skilled in the art, including but not limited to welding, stitching, bonding, and adhesives. In an embodiment, stents may be sutured to the graft body. In general, stents for use in accordance with embodiments of the present invention typically comprise a plurality of apertures or open spaces between metallic filaments (including fibres and wires), segments or regions. Typical structures include: an open-mesh network comprising one or more knitted, woven or braided metallic filaments; an interconnected network of articulable segments; a coiled or helical structure comprising one or more metallic filaments; and a patterned tubular metallic sheet (e.g., a laser cut tube).

In an embodiment, stents are located distal and proximal to the graft body. For example, as shown in Figure 1, stent 36 is located on the proximal end of the graft body 12 and stent 54 is disposed at the distal end. Stents located distally and proximally to a graft body provide structure and rigidity to the graft body. Additionally, proximal and distal stents may seal against the main vessel wall to prevent leakage around a branch vessel following perforation.

As shown in Figure 2, stents are located at the proximal and distal ends of the graft body. Stents may seal against the main vessel wall 122 to prevent undesirable fluid leakage, for example by reducing blood leakage into an aneurysmal sac 121 spanned by an implanted graft body. Additional stents may further aid in sealing against the vessel wall 122 to prevent undesirable fluid leakage into the aneurysmal sac 121.

The stents may be self-expanding or balloon-expandable, and may be deployed according to conventional methodology, such as by an inflatable balloon catheter, by a self-deployment mechanism (after release from a catheter), or by other appropriate means. The stents may be bifurcated, configured for any blood vessel including coronary arteries and peripheral arteries (e.g., renal, superficial femoral, carotid, and the like), a urethral stent, a biliary stent, a tracheal stent, a gastrointestinal stent, or an oesophageal stent, for example. Desirably, the stent is a vascular stent such as the commercially available Gianturco-Roubin FLEX-STENT®, GRII™, SUPRA-G, or V FLEX coronary stents from Cook Incorporated (Bloomington, IN).

The stents may be made of one or more suitable biocompatible materials such as stainless steel, Nitinol, MP35N, gold, tantalum, platinum or platinum irdium, niobium, tungsten, iconel, ceramic, nickel, titanium, stainless steel/titanium composite, cobalt, chromium, cobalt/chromium alloys, magnesium, aluminum, or other biocompatible metals and/or composites or alloys such as carbon or carbon fibre, cellulose acetate, cellulose nitrate, silicone, cross-linked polyvinyl alcohol (PVA) hydrogel, cross-linked PVA hydrogel foam, polyurethane, polyamide, styrene isobutylene-styrene block copolymer (Kraton), polyethylene teraphthalate, polyester, polyorthoester, polyanhydride, polyether sulphone, polycarbonate, polypropylene, high molecular weight polyethylene, polytetrafluoroethylene, or other biocompatible polymeric material, or mixture of copolymers thereof; polyesters such as, polylactic acid, polyglycolic acid or copolymers thereof, a polyanhydride, polycaprolactone, polyhydroxybutyrate valerate or other biodegradable polymer, or mixtures or copolymers thereof; extracellular matrix components, proteins, collagen, fibrin or other therapeutic agent, or mixtures thereof. Desirably, the stents comprise stainless steel or Nitinol.

### Radiopacity

The graft body may be marked for radiographic visualisation to facilitate precise alignment within the aortic artery with the particular branch anatomical conduit (e.g., carotid, innominate, subclavian, intercostal, superior mesenteric, celiac, renal, iliac, hypogastric, or visceral vessels). Radiopaque portions of the graft body would be seen by remote imaging methods including X-ray, ultrasound, magnetic resonance imaging and the like, or by detecting a signal from or corresponding to the marker.

In other embodiments, the delivery device can comprise indicia relating to the orientation of the frame within the body vessel. In other embodiments, indicia can be located, for example, on a portion of a delivery catheter that can be correlated to the location of the prosthesis within a body vessel.

Radiopaque materials may be added to the graft body by any fabrication method or absorbed into or sprayed onto the surface of part or all of the graft. The degree of radiopacity contrast can be altered by implant content. Common radiopaque materials include barium sulphate, bismuth subcarbonate, and zirconium dioxide. Other radiopaque elements include: cadmium, tungsten, gold, tantalum, bismuth, platium, iridium, and rhodium. Radiopacity is typically determined by fluoroscope or X-ray film.

### Attachment of Graft Device in Body Vessel

Prostheses according to the present invention may optionally include supplemental attachment means such as anchoring members, suturing, stapling, searing, bonding, gluing, bioadhesives, or otherwise adhering the medical device to the vessel wall or combinations thereof. For example, the graft body may be secured in place with one or more anchoring devices.

The art provides a wide variety of structural features that are acceptable for use in medical devices as anchoring members, and any suitable structural feature can be used. For example, individual barbs may be used to implant the graft body into a body vessel. The barbs may be secured to the graft body by any means known to one skilled in the art, including but not limited to welding to included stents, stitching, bonding, and adhesives. Desirably, barbs may be attached to stents included in the prosthesis. In some embodiments, the number, arrangement, and configuration of barbs can vary according to design preference and the clinical use of the graft body. The barbs can have any suitable shape, including points or "fish hook"-like configurations. The barbs may or may not penetrate the vessel wall, depending on their design and other factors.

Alternatively or in addition to anchoring members, bioadhesives may be used for attachment. Bioadhesive may be included in any suitable part of the prosthesis. Preferably, the bioadhesive is attached to the abluminal surface of the graft body. Selection of the type of bioadhesive, the portions of the prosthesis comprising the bioadhesive, and the manner of attaching the bioadhesive to the prosthesis can be chosen to perform a desired function upon implantation. For example, the bioadhesive can be selected to promote increased affinity of the desired portion of prosthesis to the section of the body vessel against which it is urged.

Bioadhesives for use in conjunction with the present invention include any suitable bioadhesives known to those of ordinary skill in the art. For example, appropriate bioadhesives include, but are not limited to, the following: (1) cyanoacrylates such as ethyl cyanoacrylate, butyl cyanoacrylate, octyl cyanoacrylate, and hexyl cyanoacrylate; (2) fibrinogen, with or without thrombin, fibrin, fibropectin, elastin, and laminin; (3) mussel adhesive protein, chitosan, prolamine gel and transforming growth factor beta (TGF-β); (4) polysaccharides such as acacia, carboxymethyl-cellulose, dextran, hyaluronic acid, hydroxypropyl-cellulose, hydroxypropyl-methylcellulose, karaya gum, pectin, starch, alginates, and tragacanth; (5) polyacrylic acid, polycarbophil, modified hypromellose, gelatin, polyvinyl-pylindone, polyvinylalcohol, polyethylene glycol, polyethylene oxide, aldehyde relative multifunctional chemicals, maleic anhydride co-polymers, and polypeptides; and (6) any bioabsorbable and biostable polymer derivatives with sticky molecules such as arginine, glycine, and aspartic acid, and copolymers.

Furthermore, commercially available bioadhesives that may be used in the present invention include, but are not limited to: FOCALSEAL^{®} (biodegradable eosin-PEG-lactide hydrogel requiring photopolymerisation with Xenon light wand) produced by Focal; BERIPLAST^{®} produced by Adventis-Bering; VIVOSTAT^{®} produced by ConvaTec (Bristol-Meyers-Squibb); SEALAGEN^{™} produced by Baxter; FIBRX^{®} (containing virally inactivated human fibrinogen and inhibited-human thrombin) produced by CryoLife; TISSEEL^{®} (fibrin glue composed of plasma derivatives from the last stages in the natural coagulation pathway where soluble fibrinogen is converted into a solid fibrin) and TISSUCOL^{®} produced by Baxter; QUIXIL^{®} (Biological Active Component and Thrombin) produced by Omrix Biopharm; a PEG-collagen conjugate produced by Cohesion (Collagen); HYSTOACRYL^{®} BLUE (ENBUCRILATE) (cyanoacrylate) produced by Davis & Geck; NEXACRYL^{™} (N-butyl cyanoacrylate), NEXABOND^{™}, NEXABOND^{™} S/C, and TRAUMASEAL^{™} (product based on cyanoacrylate) produced by Closure Medical (TriPoint Medical); DERMABOND^{®} which consists of 2-octyl cyanoacrylate produced as DERMABOND^{®} by (Ethicon); TISSUEGLU^{®} produced by Medi-West Pharma; and VETBOND^{®} which consists of n-butyl cyanoacrylate produced by 3M.

### Bioactive Agents

Optionally, the graft body can include at least one bioactive agent. The bioactive agent can be included in any suitable part of the prosthesis. The bioactive materials can be attached to the prosthesis in any suitable manner. For example, a bioactive agent may be sprayed onto the graft body material, or stents may be dipped in bioactive agent. Selection of the type of bioactive agent, the portions of the prosthesis comprising the bioactive agent, and the manner of attaching the bioactive agent to the prosthesis can be chosen to perform a desired function upon implantation. For example, the bioactive material can be selected to treat indications such as coronary artery angioplasty, renal artery angioplasty, carotid artery surgery, renal dialysis fistulae stenosis, or vascular graft stenosis.

The bioactive agent can be selected to perform one or more desired biological functions. For example, the abluminal surface of the graft body can comprise a bioactive selected to promote the ingrowth of tissue from the interior wall of a body vessel, such as a growth factor. An anti-angiogenic or anti-neoplastic bioactive such as paclitaxel, sirolimus, or a rapamycin analogue, or a metalloproteinase inhibitor such as batimastat can be incorporated in or coated on the prosthesis to mitigate or prevent undesired conditions in the vessel wall, such as restenosis. Many other types of bioactive agents can be incorporated in the prosthesis.

Bioactive materials for use in biocompatible coatings include those suitable for coating an implantable medical device. The bioactive agent can include, for example, one or more of the following: antiproliferative agents (sirolimus, paclitaxel, actinomycin D, cyclosporine), immunomodulating drugs (tacrolimus, dexamethasone), metalloproteinase inhibitors (such as batimastat), antisclerosing agents (such as collagenases, halofuginone), prohealing drugs (nitric oxide donors, estradiols), mast cell inhibitors and molecular interventional bioactive agents such as c-myc antisense compounds, thromboresistant agents, thrombolytic agents, antibiotic agents, anti-tumour agents, antiviral agents, anti-angiogenic agents, angiogenic agents, anti-mitotic agents, anti-inflammatory agents, angiostatin agents, endostatin agents, cell cycle regulating agents, genetic agents, including hormones such as oestrogen, their homologues, derivatives, fragments, and pharmaceutical salts, and combinations thereof. Other useful bioactive agents include, for example, viral vectors and growth hormones such as Fibroblast Growth Factor and TGF-β.

Further examples of antithrombotic bioactive agents include anticoagulants such as heparin, low molecular weight heparin, covalent heparin, synthetic heparin salts, coumadin, bivalirudin (hirulog), hirudin, argatroban, ximelagatran, dabigatran, dabigatran etexilate, D-phenalanyl-L-poly-L-arginyl, chloromethyl ketone, dalteparin, enoxaparin, nadroparin, danaparoid, vapiprost, dextran, dipyridamole, omega-3 fatty acids, vitronectin receptor antagonists, DX-9065a, CI-1083, JTV-803, razaxaban, BAY 59-7939, and LY-51,7717; antiplatelets such as eftibatide, tirofiban, orbofiban, lotrafiban, abciximab, aspirin, ticlopidine, clopidogrel, cilostazol, dipyradimole, nitric oxide sources such as sodium nitroprussiate, nitroglycerin, S-nitroso and N-nitroso compounds; fibrinolytics such as alfimeprase, alteplase, anistreplase, reteplase, lanoteplase, monteplase, tenecteplase, urokinase, streptokinase, or phospholipid encapsulated microbubbles; and other bioactive agents such as endothelial progenitor cells or endothelial cells.

### Delivery of Graft Device

The graft device can be configured for delivery to a body vessel. For example, a prosthesis comprising a graft body and stents according to embodiments of the present invention can be compressed to a delivery configuration within a retaining sheath that is part of a delivery system, such as a catheter-based system. Upon delivery, the prosthesis can be expanded, for example, by inflating a balloon from inside the stents. The delivery configuration can be maintained prior to deployment of the prosthesis by any suitable means, including a sheath, a suture, a tube or other restraining material around all or part of the compressed prosthesis, or other methods.

Prostheses can be deployed in a body vessel by means appropriate to their design. Prostheses of the present invention can be adapted for deployment using conventional methods known in the art and employing percutaneous transluminal catheter devices. The prostheses are designed for deployment by any of a variety of *in situ* expansion means.

In an embodiment, a prosthesis comprising self-expanding stents and a graft body may be mounted onto a catheter that holds the prosthesis as it is delivered through the body lumen and then releases the prosthesis and allows it to self-expand into contact with the body lumen. This deployment is effected after the prosthesis has been introduced percutaneously, transported transluminally and positioned at a desired location by means of the catheter. The self-expanding prosthesis may be deployed according to well-known deployment techniques for self-expanding medical devices. For example, the prosthesis may be positioned at the distal end of a catheter with a removable sheath or sleeve placed over the prosthetic valve to hold the prosthesis in a contracted state with a relatively small diameter. The prosthesis may then be implanted at the point of treatment by advancing the catheter over a guide wire to the location of the lesion, aligning graft body within the aortic arterty and with any branch vessels, and then withdrawing the sleeve from over the prosthesis. The stent graft will automatically expand and exert pressure on the wall of the blood vessel at the site of treatment. The catheter, sleeve, and guide wire may then be removed from the patient.

In some embodiments, a bioabsorbable suture or sheath can be used to maintain a self-expanding stent graft in a compressed configuration both prior to and after deployment. As the bioabsorbable sheath or suture is degraded by the body after deployment, the prosthesis can expand within the body vessel. In some embodiments, a portion of the prosthesis can be restrained with a bioabsorbable material and another portion allowed to expand immediately upon implantation. For example, a self-expanding stent graft can be partially restrained by a bioabsorbable material upon deployment and later expand as the bioabsorbable material is absorbed.

In another embodiment, a stent graft may be first positioned to surround a portion of an inflatable balloon catheter. The prosthesis, with the balloon catheter inside is configured at a first, collapsed diameter. The prosthesis and the inflatable balloon are percutaneously introduced into a body vessel, following a previously positioned guide wire. For example, in rapid exchange, a rapid exchange prosthesis delivery balloon catheter allows exchange from a balloon angioplasty catheter to a prosthesis delivery catheter without the need to replace the angioplasty catheter guide wire with an exchange-length wire guide before exchanging the catheters. The prosthesis may be tracked by a fluoroscope, until the balloon portion and associated prosthesis are positioned within the body passageway at the point where the prosthesis is to be placed. Thereafter, the balloon is inflated and the prosthesis is expanded by the balloon portion from the collapsed diameter to a second expanded diameter. After the prosthesis has been expanded to the desired final expanded diameter, the balloon is deflated, and the catheter may be withdrawn, leaving the prosthesis in place. The prosthesis may be covered by a removable sheath during delivery to protect both the prosthesis and the vessels.

While the present invention has been described in terms of preferred embodiments, it will be understood, of course, that the invention is not limited thereto since modifications within the scope of the claims may be made by those skilled in the art, particularly in light of the foregoing teachings.

## Claims

1. An implantable graft device (10) having a composite weave graft, the device including:
a graft body (12) forming a lumen (20) defining a longitudinal axis and including a proximal end (21) and a distal end (22), the graft body including a composite of warp yarns (30) including low denier yarns and weft yarns (32) including polymeric elastic yarns, configured for low profile delivery and radial elongation relative to the longitudinal axis during use, the graft body having a first portion (40) and a second portion (42) extending from the first portion, the first portion including at least one expandable stent (43) radially attached thereto for support and the second portion having corrugations (46) for enhanced kink resistance;
an anchor portion (13) extending from the proximal end (21) of the graft body (12), the anchor portion having a first woven portion (50) and a barb stent (52) attached thereto for reduced migration of the graft device, the first woven portion including woven yam, the low denier yam of the graft body having a smaller diameter than the yam of the anchor portion; and
an end portion (14) extending from the distal end of the graft body, the end portion having a second woven portion (56) and an expandable stent (43) attached thereto, the second woven portion being comprised of woven yarn, **characterised in that** the low denier yam of the graft body has a smaller diameter than the yam of the end portion.

2. A device (10) as claimed in claim 1, wherein the first woven portion (50) includes an inner side (16), the barb stent (52) being attached to the inner side of the first woven portion.

3. A device (10) as claimed in claim 2, wherein the barb stent (52) includes a plurality of loops attached to the first woven portion (50).

4. A device (10) as claimed in claim 1, 2 or 3, wherein the graft body (12) comprises an inner side (16) and an outer side (18), at least one expandable stent (43) being attached to the inner side of the graft body.

5. A device (10) as claimed in any preceding claim, wherein the low denier yam comprises polyester.

6. A device as claimed in claim 5, wherein the low denier yam has a density between about 0.5 and 1.5 denier.

7. A method for making an implantable graft device (10) having a composite weave graft, the method including:
forming a graft body (12) having a lumen (20) defining a longitudinal axis and including a proximal end (21) and a distal end (22) defining a woven fabric having warp yarns (30) including low denier yarns aligned in a first direction and weft yarns (32) including elastic yarns aligned in a second direction and interwoven with the warp yarns, the woven fabric being configured for low profile delivery and radial elongation relative to the longitudinal axis during use, the graft body having a first portion (40) and a second portion (42) extending from the first portion;
attaching at least one expandable stent (43) radially about the first portion for support;
forming corrugations (46) on the second portion for enhanced kink resistance;
attaching an anchor portion (13) to the proximal end (21) and extending therefrom, the anchor portion having a first woven portion (50) including woven yam, the reduced diameter yam of the graft body (12) having a smaller diameter than the yam of the first woven portion, the first woven portion having a barb stent (52) attached thereto for reduced migration of the graft device; and
attaching an end portion (14) to the distal end (22) and extending therefrom, the end portion having a second woven portion (56) and an expandable stent (43) attached thereto, the second woven portion being comprised of woven yarn, and **characterised in that** the reduced diameter yam of the graft body (12) has a smaller diameter than the yam of the end portion.

8. A method as claimed in claim 7, wherein the reduced diameter yam is low denier polyester yarn.

9. A method as claimed in claim 8, wherein the low denier yarn has a density between about 0.5 and 1.5 denier.

10. A delivery system including a device (10) or prosthesis (10) as claimed in any preceding claim.

## Patentansprüche

1. Implantierbare Graftprothesenvorrichtung (10) mit einer gewebten Verbundgraftprothese, wobei die Vorrichtung Folgendes umfasst:
einen Graftprothesenkörper (12), der ein Lumen (20) bildet, das eine Längsachse definiert und ein proximales Ende (21) und ein distales Ende (22) aufweist, wobei der Graftprothesenkörper einen Verbund aus Kettgarnen (30) mit Garnen mit niedrigem Feinheitsgrad und Schussgarnen (32) mit elastischen Polymergarnen aufweist, die für niedrigprofilige Abgabe und radiale Dehnung relativ zur Längsachse im Gebrauch konfiguriert sind, wobei der Graftprothesenkörper einen ersten Abschnitt (40) und einen sich vom ersten Abschnitt aus erstreckenden zweiten Abschnitt (42) aufweist, wobei der erste Abschnitt zumindest einen expandierbaren Stent (43) aufweist, der zur Unterstützung radial daran befestigt ist und der zweite Abschnitt Riffelungen (46) für verbesserte Knickfestigkeit aufweist;
einen Ankerabschnitt (13), der sich vom proximalen Ende (21) des Graftprothesenkörpers (12) erstreckt, wobei der Ankerabschnitt einen ersten gewebten Abschnitt (50) und einen daran befestigten Widerhakenstent (52) zur Verringerung des Verrutschens der Graftprothesenvorrichtung aufweist, wobei der erste gewebte Abschnitt gewebtes Garn aufweist, wo bei das Garn mit niedrigem Feinheitsgrad des Graftprothesenkörpers einen kleineren Durchmesser aufweist als das Garn des Ankerabschnitts; und
einen Endabschnitt (14), der sich vom distalen Ende des Graftprothesenkörpers aus erstreckt, wobei der Endabschnitt einen zweiten gewebten Abschnitt (56) und
einen daran befestigten expandierbaren Stent (43) aufweist, wobei der zweite gewebte Abschnitt aus gewebten Garn besteht, **dadurch gekennzeichnet, dass** das Garn mit niedrigem Feinheitsgrad des Graftprothesenkörpers einen kleineren Durchmesser aufweist als das Garn des Endabschnitts.

2. Vorrichtung (10) nach Anspruch 1, worin der erste gewebte Abschnitt (50) einen Innenseite (16) aufweist, wobei der Widerhakenstent (52) an der Innenseite des ersten gewebten Abschnitts befestigt ist.

3. Vorrichtung (10) nach Anspruch 2, worin der Widerhakenstent (52) eine Vielzahl von am ersten gewebten Abschnitt (50) befestigten Schlaufen aufweist.

4. Vorrichtung (10) nach Anspruch 1, 2 oder 3, worin der Graftprothesenkörper (12) eine Innenseite (16) und eine Außenseite (18) umfasst, wobei zumindest ein expandierbarer Stent (43) an der Innenseite des Graftprothesenkörpers befestigt ist.

5. Vorrichtung (10) nach einem der vorhergehenden Ansprüche, worin das Garn mit niedrigem Feinheitsgrad Polyester umfasst.

6. Vorrichtung nach Anspruch 5, worin das Garn mit niedrigem Feinheitsgrad eine Dichte zwischen ca. 0,5 und 1,5 Denier aufweist.

7. Verfahren zur Herstellung einer implantierbaren Graftprothesenvorrichtung (10) mit einer gewebten Verbundgraftprothese, wobei das Verfahren Folgendes umfasst:
Bildung eines Graftprothesenkörpers (12), der ein Lumen (20) aufweist, das eine Längsachse definiert und ein proximales Ende (21) und ein distales Ende (22) aufweist und ein Gewebe mit Kettgarnen (30) mit Garnen mit niedrigem Feinheitsgrad, die in einer ersten Richtung ausgerichtet sind, und Schussgarnen (32) mit elastischen Garnen, die in einer zweiten Richtung ausgerichtet sind und mit den Kettgarnen verwebt sind, definiert, wobei das Gewebe zur niederprofiligen Abgabe und radialen Dehnung relativ zur Längsachse im Gebrauch konfiguriert ist, wobei der Graftprothesenkörper einen ersten Abschnitt (40) und einen sich vom ersten Abschnitt aus erstreckenden zweiten Abschnitt (42) aufweist;
Befestigung zumindest eines expandierbaren Stents (43) radial um den ersten Abschnitt zur Unterstützung;
Bildung von Riffelungen (46) auf dem zweiten Abschnitt für verbesserte Knickfestigkeit;
Befestigung eines Ankerabschnitts (13) am proximalen Ende (21), der sich davon erstreckt, wobei der Ankerabschnitt einen ersten gewebten Abschnitt (50) mit gewebtem Garn aufweist, wobei das Garn mit kleinerem Durchmesser des Graftprothesenkörpers (12) einen kleineren Durchmesser aufweist als das Garn des ersten gewebten Abschnitts, wobei der erste gewebte Abschnitt einen daran befestigten Widerhakenstent (52) zur Verringerung des Verrutschens der Graftprothesenvorrichtung aufweist; und
Befestigung eines Endabschnitts (14) am distalen Ende (22), der sich davon erstreckt, wobei der Endabschnitt einen zweiten gewebten Abschnitt (56) und einen daran befestigten expandierbaren Stent (43) aufweist, wobei der zweite gewebte Abschnitt aus gewebten Garn besteht,
**dadurch gekennzeichnet, dass** das Garn mit kleinerem Durchmesser des Graftprothesenkörpers (12) einen kleineren Durchmesser aufweist als das Garn des Endabschnitts.

8. Verfahren nach Anspruch 7, worin das Garn mit kleinerem Durchmesser Polyestergarn mit niedrigem Feinheitsgrad ist.

9. Verfahren nach Anspruch 8, worin das Garn mit niedrigem Feinheitsgrad eine Dichte zwischen ca. 0,5 und 1,5 Denier aufweist.

10. Abgabesystem mit einer Vorrichtung (10) oder einer Prothese (10) nach einem der vorhergehenden Ansprüche.

## Revendications

1. Dispositif (10) de greffe implantable pourvu d'une greffe de tissage composite, le dispositif comprenant :
un corps (12) de greffe formant une lumière (20) délimitant un axe longitudinal et comprenant une extrémité (21) proximale et une extrémité (22) distale, le corps de greffe comprenant un composite de fils (30) de chaîne comprenant des fils de faible denier et de fils (32) de trame comprenant des fils polymère élastiques, configuré pour une mise en place à plat et un allongement radial par rapport à l'axe longitudinal pendant l'utilisation, le corps de greffe présentant une première partie (40) et une seconde partie (42) s'étendant depuis la première partie, la première partie comprenant au moins un stent (43) extensible attaché radialement à celle-ci pour un support et la seconde partie comportant des ondulations (46) pour une résistance à une formation de vrilles améliorée ;
une partie (13) ancre s'étendant depuis l'extrémité (21) proximale du corps (12) de greffe, la partie ancre présentant une première partie (50) tissée et un stent (52) à barbillons attaché à celle-ci pour réduire la migration du dispositif de greffe, la première partie tissée comprenant un fil tissé, le fil de faible denier du corps de greffe ayant un diamètre plus petit que le fil de la partie ancre ; et
une partie (14) d'extrémité s'étendant depuis l'extrémité distale du corps de greffe, la partie extrémité présentant une seconde partie (56) tissée et un stent (43) extensible attaché à celle-ci, la seconde partie tissée comprenant un fil tissé, **caractérisé en ce que** le fil de faible denier du corps de greffe a un diamètre plus petit que le fil de la partie extrémité.

2. Dispositif (10) selon la revendication 1, dans lequel la première partie (50) tissée comprend une face (16) interne, le stent (52) à barbillons étant attaché à la face interne de la première partie tissée.

3. Dispositif (10) selon la revendication 2, dans lequel le stent (52) à barbillons comprend une pluralité de boucles attachées à la première partie (50) tissée.

4. Dispositif (10) selon la revendication 1, 2, ou 3, dans lequel le corps (12) de greffe comporte une face (16) interne et une face (18) externe, au moins un stent (43) extensible étant attaché à la face interne du corps de greffe.

5. Dispositif (10) selon l'une quelconque des revendications précédentes, dans lequel le fil de faible denier comporte du polyester.

6. Dispositif selon la revendication 5, dans lequel le fil de faible denier a une densité se situant entre 0,5 et 1,5 denier.

7. Procédé pour fabriquer un dispositif (10) de greffe implantable pourvu d'une greffe de tissage composite, le procédé consistant à :
former un corps (12) de greffe présentant une lumière (20) délimitant un axe longitudinal et comprenant une extrémité (21) proximale et une extrémité (22) distale délimitant un tissu tissé possédant des fils (30) de chaîne comprenant des fils de faible denier alignés dans une première direction et des fils (32) de trame comprenant des fils élastiques alignés dans une seconde direction et entrelacés avec les fils de chaîne, le tissu tissé étant configuré pour une mise en place à plat et un allongement radial relatif par rapport à l'axe longitudinal pendant l'utilisation, le corps de greffe présentant une première partie (40) et une seconde partie (42) s'étendant depuis la première partie ;
attacher au moins un stent (43) extensible radialement autour de la première partie pour un support ;
former des ondulations (46) sur la seconde partie pour une résistance à une formation de vrilles améliorée ;
attacher une partie (13) ancre à l'extrémité (21) proximale et s'étendant depuis celle-ci, la partie ancre présentant une première partie (50) tissée comprenant du fil tissé, le fil à diamètre réduit du corps (12) de greffe ayant un diamètre plus petit que le fil de la première partie tissée, la première partie tissée étant munie d'un stent (52) à barbillons attaché à celle-ci pour réduire la migration du dispositif de greffe ; et
attacher une partie (14) extrémité à l'extrémité (22) distale et s'étendant depuis celle-ci, la partie extrémité présentant une seconde partie (56) tissée et un stent (43) extensible attaché à celle-ci, la seconde partie tissée étant constituée de fil tissé, et **caractérisé en ce que** le fil au diamètre réduit du corps (12) de greffe a un diamètre plus petit que le fil de la partie extrémité.

8. Procédé selon la revendication 7, dans lequel le fil au diamètre réduit est un fil polyester de faible denier.

9. Procédé selon la revendication 8, dans lequel le fil de faible denier a une densité se situant entre environ 0,5 et 1,5 denier.

10. Système de mise en place comprenant un dispositif (10) ou prothèse (10) selon l'une quelconque des revendications précédentes.
